(19) Europäisches Patentamt
European Patent Office
Office européen des brevets

(11) Veröffentlichungsnummer : **0 080 118**
**B1**

(12) # EUROPÄISCHE PATENTSCHRIFT

(45) Veröffentlichungstag der Patentschrift : **21.10.87**

(51) Int. Cl.⁴ : **C 07 D 317/22**, C 07 C 43/303, C 07 C 41/54

(21) Anmeldenummer : **82110413.0**

(22) Anmeldetag : **11.11.82**

(54) **Verfahren zur Herstellung von Malondialdehydtetraalkylacetalen.**

(30) Priorität : **19.11.81 DE 3145709**

(43) Veröffentlichungstag der Anmeldung : **01.06.83 Patentblatt 83/22**

(45) Bekanntmachung des Hinweises auf die Patenterteilung : **21.10.87 Patentblatt 87/43**

(84) Benannte Vertragsstaaten : **BE CH DE FR GB IT LI**

(56) Entgegenhaltungen :
**EP-A- 0 058 928**
**US-A- 2 459 076**
**US-A- 2 823 226**
**HOUBEN-WEYL "Methoden der organischen Chemie" 4. Auflage, GEORG THIEME VERLAG, Stuttgart, 1965 Band 6/3, Seite 248**
**HOUBEN-WEYL "Methoden der organischen Chemie" 4. Auflage, GEORG THIEME VERLAG, Stuttgart, 1965 Band 6/3, Seite 311**
**Chemical Abstracts Band 78,Nr. 13, 2. April 1973 Columbus, Ohio, USA J.M. KLIEGMAN et al. "Glyoxal derivatives" Seite 429, Spalte 2, Abstract Nr. 84302w & J.Org. Chem. Band 38, Nr. 3, 1973, Seiten 556-560**
**Chemical Abstracts Band 96,Nr. 23, 7. Juni 1982 Columbus, Ohio, USA B.A. TROFIMOV et al. "Dihydropyran derivatives. VI. Reaction of functional derivatives of 3,4-dihydropyran with alcohols" Seite 661, Spalten 1,2, Abstract Nr. 199556w**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Eckhardt, Heinz, Dr.**
**Hessheimer Strasse 50**
**D-6710 Frankenthal (DE)**
Erfinder : **Halbritter, Klaus, Dr.**
**Schwarzwaldstrasse 19**
**D-6800 Mannheim 1 (DE)**
Erfinder : **Rohr, Wolfgang, Dr.**
**In der Dreispitz 13**
**D-6706 Wachenheim (DE)**

**EP 0 080 118 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Malondialdehydtetraalkylacetalen durch Umsetzung von Alkylformiaten, Oxiranen und Alkylvinylethern in Gegenwart von Halogeniden des Bors und/oder Antimons (V).

Die Umsetzung von Orthoameisensäureestern mit Vinylethern bzw. Vinylestern in Gegenwart saurer Katalysatoren wie $BF_3$ zu Malondialdehydtetraalkylacetal ist aus Houben-Weyl, Methoden der Organischen Chemie, Bd. VI/3 (1965), S. 248 (1) und der US-PS-2 459 976 (3) bekannt. Beide Schriften lehren, daß zur Erzielung guter Ausbeuten 2 und mehr Mol Orthoester pro Mol ungesättigte Verbindung benötigt werden. (3) zeigt weiterhin, daß mit $BF_3$ als Katalysator vergleichsweise schlechte Ausbeuten erhalten werden (s. Beispiel 5).

Die Herstellung cyclischer Orthocarbonsäureester durch Anlagerung von 1,2-Epoxiden an Ameisensäureester in Gegenwart von Bortrifluorid ist in Houben-Weyl, Methoden der Organischen Chemie, Bd. VI/3 (1965), S. 311 (2) beschrieben. Die in (2) angegebene Arbeitsvorschrift ist der Dissertation von K. Bodenbenner, Marburg 1953 (2a), Seiten 56-59, entnommen. (2a) zeigt sowohl die Umsetzung mit Ethylenoxid ($2a_1$), die (2) nicht näher beschreibt, als auch mit Epichlorhydrin ($2a_2$). Um die in (2) zitierten Ausbeuten zu erhalten, sind Reaktionszeiten von mehr als 1 Tag ($2a_1$) bzw. 2 Tagen ($2a_2$) erforderlich. Der Fachmann mußte somit schon aufgrund der unwirtschaftlichen Reaktionszeiten ein gewisses technisches Vorurteil gegen die Übertragung dieser Arbeitsweise auf andere Ausgangsstoffe, z. B. die erfindungsgemäßen alkylsubstituierten Epoxide, hegen. Betrachtet der Fachmann die Ausbeuten von (2), ($2a_1$) und ($2a_2$), so verstärkt sich sein Vorurteil : Selbst im Fall von (2) und der längsten Reaktionszeit von mehr als 2 Tagen erhält man lediglich eine Ausbeute von 59,6 % der Theorie, auf der anderen Seite aber 31,8 % aus Epichlorhydrin gebildete Polymere. Es zeigt sich so eine für ein großtechnisches Verfahren nicht akzeptable Bildung größerer Mengen an schwer abtrennbaren, harzigen Rückständen. Die Umsetzung verläuft noch schlechter, wenn man vom Chlormethyl-substituierten zum unsubstituierten Ethylenoxid übergeht. (2) zeigt die mäßige Ausbeute von ca. 25 % ($2a_2$) zeigt darüber hinaus (Seite 57), daß selbst diese Ausbeute aus unreinem Endstoff besteht, da nämlich der rohe Endstoff in 2 Fraktionen (11,5 % und 14,2 % der Theorie) in einem Temperaturintervall von 139-150 °C abgetrennt wird.

Es wurde nun ein Verfahren zur Herstellung von Gemischen der Verbindungen (Ia), (Ib) und (Ic)

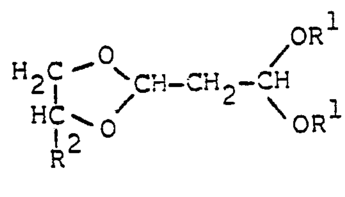

(Ia)

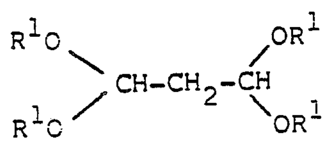

(Ib)

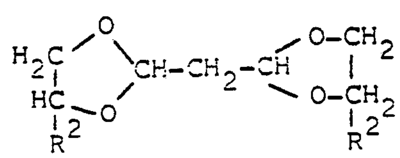

(Ic)

worin die einzelnen Reste $R^1$ gleich oder verschieden sein können und einen Alkylrest mit 1 bis 6 Kohlenstoffatomen bedeuten und $R^2$ Wasserstoff oder einen Alkylrest mit 1 bis 6 Kohlenstoffatomen darstellt, gefunden, das dadurch gekennzeichnet ist, daß man Alkylformiate der Formel (II)

$$HCOOR^1 \quad (II)$$

in Gegenwart katalytischer Mengen von Halogeniden des Bors und/oder Antimons (V) zunächst mit Oxiranen der Formel (III)

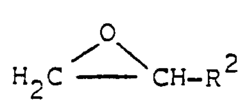

(III)

in der $R^2$ die vorgenannte Bedeutung hat, und dann in situ mit Alkylvinylethern der Formel (IV)

$$CH_2{=}CH{-}OR^1 \qquad (IV)$$

in der $R^1$ die vorgenannte Bedeutung hat, umsetzt, wobei die Ausgangsstoffe (II) und/oder (III) in einem Verhältnis von 0,5 bis 3,0 mol pro Mol Ausgangsstoff (IV) verwendet werden.

Die Umsetzung kann für den Fall der Verwendung von Methylformiat, 2-Methyloxiran und Methylvinylether durch die folgenden Formeln beschrieben werden :

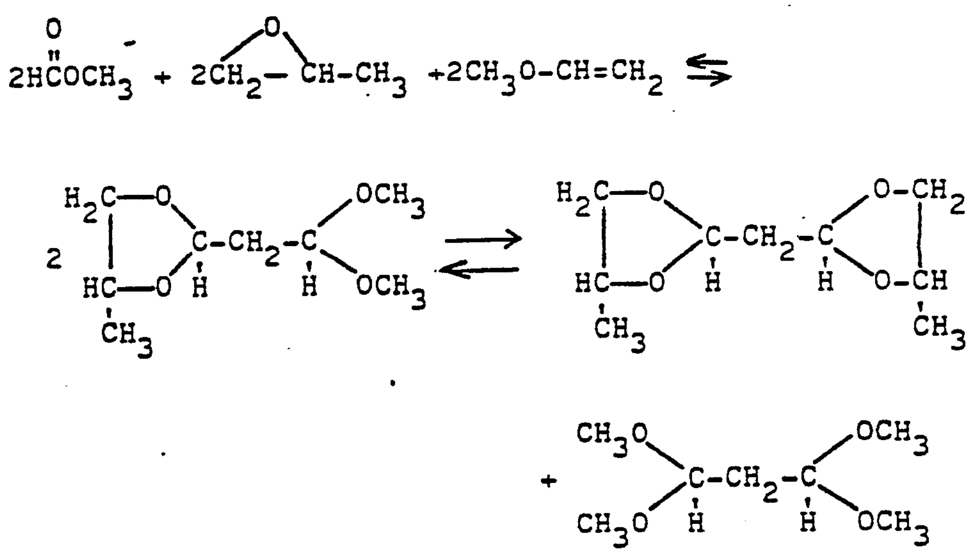

Im Vergleich zu dem bekannten Verfahren liefert das Verfahren nach der Erfindung auf einfacherem und wirtschaftlicherem Wege Malondialdehydtetraalkylacetale in hoher Ausbeute und Reinheit.

Ein weiterer Vorteil ist, daß die Verwendung und die Handhabung der hydrolyseempfindlichen Orthoformiate, deren Herstellung mit einem hohen Salzanfall, dem Umgang mit Ausgangsstoffen hoher Toxizität und der Notwendigkeit korrosionsfester Apparaturen verbunden ist, vermieden wird. Die erfindungsgemäße Herstellung von Malondialdehydtetraalkylacetalen Ia bis Ic aus einfachen Alkylformiaten hat in vorteilhafter Weise alle diese Nachteile der Orthoformiatherstellung nicht.

Man kann die Ausgangsstoffe (II), (III) und (IV) in stöchiometrischer Menge oder im Überschuß jeder Komponente zur anderen, zweckmäßig in einem Verhältnis von 0,5 bis 3,0, vorteilhaft von 1,0 bis 2,0 Mol Ausgangsstoff (III) und/oder von 0,5 bis 3,0, vorteilhaft von 1,0 bis 2,0 Mol Ausgangsstoff (II) je Mol Ausgangsstoff (IV) umsetzen. Ausgangsstoffe (II), (III), (IV) und dementsprechend Endstoffe (Ia) bis (Ic) sind solche, in deren Formeln die einzelnen Reste $R^1$ gleich oder verschieden sind und jeweils einen Alkylrest mit 1 bis 6 Kohlenstoffatomen bedeuten und worin $R^2$ ein Wasserstoffatomen oder einen Alkylrest mit 1 bis 3 Kohlenstoffatomen bedeutet.

Zum Beispiel können folgende Alkylformiate (II) verwendet werden : Methyl-, Ethyl-, Propyl-, Isopropyl-, Butyl-, Isobutyl-, sek.-Butyl-, tert.-Butyl-, Pentyl-, Hexyl-formiat.

Folgende Ausgangsstoffe (III) sind geeignet : Oxiran, 2-Methyl-, 2-Ethyl-, 2-Propyl-, 2-Isopropyl-, 2-Butyl-, 2-Isobutyl-, 2-sekt.-Butyl-, 2-tert.-Butyl-, 2-Pentyl-, 2-Hexyl-oxiran.

Beispielsweise kommen als Ausgangsstoffe (IV) in Frage : Methyl-, Ethyl-, Propyl-, Isopropyl-, Butyl-, Isobutyl-, sek.-Butyl- oder tert.-Butyl-vinylether.

Die als Katalysatoren dienenden Halogenide werden zweckmäßig in einer Menge von 0,05 bis 5,0, insbesondere von 0,1 bis 1,0 Gew.-% Halogenid, bezogen auf die Gewichtsmenge Ausgangsstoff (II), verwendet. Auch Anlagerungsverbindungen des Halogenids, z. B. $BF_3{-}HCOOCH_3$ und $BF_3{-}C_2H_5OC_2H_5$ können verwendet werden.

Bevorzugt ist $BF_3$ und seine Anlagerungsverbindungen. In einer bevorzugten Ausführungsform werden Bor- und Antimonhalogenide gleichzeitig aber separat mit dem Alkylformiat, und separat mit dem Alkylvinylether dem Ausgangsgemisch zugegeben. Unter den Halogeniden sind Fluoride und Chloride bevorzugt. Zur Neutralisation des Katalysators, der im Reaktionsgemisch vollständig oder teilweise gelöst ist, können übliche organische oder anorganische Basen verwendet werden (z. B. Metalloxide, -carbonate, -hydroxide, -alkoholate, Amine, Amide).

Die Umsetzung wird in der Regel bei einer Temperatur von — 80 bis + 200 °C, zweckmäßig von 0 bis 100 °C, vorteilhaft von 0 bis 70 °C, insbesondere 20 bis 70 °C, drucklos, mit Unterdruck oder unter Druck, vorteilhaft bei einem Druck von 1 bis 4 bar, kontinuierlich oder diskontinuierlich durchgeführt. Bevorzugt arbeitet man in einem Temperaturbereich, der zwischen 0 °C und dem Siedepunkt des Reaktionsgemisches liegt. Als Lösungsmittel können z. B. aromatische und aliphatische Kohlenwasserstoffe, Halogenkohlenwasserstoffe, Carbonsäureester oder Ether verwendet werden ; bevorzugt führt man die Reaktion

in überschüssigem Alkylformiat durch, wobei in dem Falle der Verwendung als Lösungsmittel zweckmäßig 5 bis 40, vorteilhaft 10 bis 20 Mol Ausgangsstoff (II) je Mol Ausgangsstoff (IV) verwendet werden.

Die Reaktion kann wie folgt durchgeführt werden : Ein Gemisch von Katalysator, Ausgansstoffe (II), (III) und (IV) und gegebenenfalls von organischen Lösungsmitteln wird während 1 bis 15 Stunden bei der Reaktionstemperatur gehalten. Bei der diskontinuierlichen Verfahrensweise wird zweckmäßig der Katalysator mit Alkylformiat und gegebenenfalls Lösungsmittel vorgelegt und das Oxiran oder eine Lösung des Oxirans in einem Lösungsmittel, das auch Alkylformiat enthalten kann, dem Reaktionsgemisch ständig zugeführt. Die hierfür benötigte Zeit kann zweckmäßig zwischen 10 und 600 Minuten, bevorzugt zwischen 15 und 150 Minuten, betragen. Anschließend kann vorteilhaft das Lösungsmittel und überschüssiges Alkylformiat durch Destillation soweit abgetrennt werden, bis die Siedetemperatur des Reaktionsgemisches zweckmäßig zwischen 50 bis 80 °C liegt : diese Destillation ist für das Verfahren nicht notwendig, aber vorteilhaft, da so eine Reinigung des rückgewonnenen Alkylformiats und des Lösungsmittels vermieden werden kann. Dem so erhaltenen Reaktionsgemisch führt man innerhalb von 1 bis 5 Stunden, bevorzugt 1 bis 2 Stunden, einen Alkylvinylether, gegebenenfalls gemeinsam mit einem Lösungsmittel, zu. Nach Ende der Reaktion wird vorteilhaft der Katalysator mit vorgenannten Basen neutralisiert und der Endstoff in üblicher Verfahrensweise, z. B. durch Destillation, isoliert. Bei der Destillation gewinnt man einen Vorlauf, der noch weiteren Endstoff enthält und vorteilhaft in einer folgenden Reaktion dem Reaktionsgemisch vor der Zugabe des Alkylvinylethers zugesetzt wird. Eine solche Verfahrensweise ermöglicht eine Erhöhung der Ausbeute.

Eine bevorzugte Ausführungsform für die kontinuierliche Verfahrensweise findet vorteilhaft in einer Kaskade von drei Reaktion statt. In den ersten werden gleichzeitig eine Lösung von einem Oxiran in einem Alkylformiat, gegebenenfalls unter Zusatz eines weiteren Lösungsmittels, und eine Lösung von $BF_3$ in einem Lösungsmittel zugeführt. Das nach einer vorgegebenen Verweilzeit, die zweckmäßig zwischen 10 und 200 Minuten, bevorzugt zwischen 10 und 30 Minuten, liegen kann, aus dem Reaktor fließende Reaktionsgemisch wird in einer Destillationsapparatur von überschüssigem Alkylformiat und Lösungsmittel befreit. Dieses Destillat wird in den ersten Reaktor zurückgeführt, während der Destillationsrückstand in den zweiten Reaktor fließt und dort mit einem Alkylvinylether vermischt wird. Nach einer vorgegebenen Verweilzeit, die hier zweckmäßig ebenfalls zwischen 10 und 200 Minuten, bevorzugt 30 bis 60 Minuten, liegen kann, wird das Reaktionsgemisch im dritten Reaktor zur Neutralisation des Katalysators mit einer der vorgenannten Basen, die vorzugsweise flüssig ist, versetzt. Der Endstoff wird nach üblichen Verfahrensweisen, z. B. Destillation, isoliert. Der Vorlauf der Destillation, der auch hier Endstoff enthält, wird zur Erhöhung der Ausbeute vorteilhaft in den zweiten Reaktor zurückgeführt. Auch hier ist es möglich, auf die oben beschriebene Destillation des Reaktionsgemisches aus dem ersten Reaktor zu verzichten und das überschüssige Alkylformiat und Lösungsmittel erst in der abschließenden Destillation abzutrennen ; es kann dann, gegebenenfalls nach einem Reinigungsprozeß, in den ersten Reaktor zurückgeführt werden.

Die nach dem Verfahren der Erfindung herstellbaren Malondialdehydtetraalkylacetale sind wertvolle Ausgangsstoffe für die Herstellung von Farbstoffen, Schädlingsbekämpfungsmitteln und Pharmazeutika. Sie können als Zwischenprodukte für die Synthese von heterocyclischen Verbindungen (z. B. Pyrazol, Isoxazol, Pyrimidin, 2-Aminopyrimidin, Pyrimidon) verwendet werden, die ihrerseits als Zwischenprodukte für Pflanzenschutzmittel, Farbstoffe und Pharmawirkstoffe verwendet werden.

Die in den folgenden Beispielen genannten Teile sind Gewichtsteile. Sie verhalten sich zu den Volumenteilen wie Kilogramm zu Liter.

## Beispiel 1

Man legt in einem Reaktor 1 000 Teile Methylformiat und einen Teil $BF_3$—$HCOOCH_3$ vor und erwärmt auf 30 °C. Dann wird innerhalb von 3,5 Stunden bei 30 bis 32 °C eine Mischung aus 100 Teilen 1,2-Propylenoxid und 100 Teilen Methylformiat zugegeben. Nach weiteren 15 Minuten versetzt man das Reaktionsgemisch mit einem Teil $BF_3 \cdot HCOOCH_3$ und gibt innerhalb von 2 Stunden bei 25 bis 32 °C eine Mischung aus 81 Teilen Methylvinylether und 50 Teilen Methylformiat zu. Anschließend wird der Katalysator mit 5 Teilen $NaOCH_3$ neutralisiert und das Reaktionsgemisch fraktioniert destilliert. Man erhält bei 55 bis 85 °C/1 mbar 171 Teile Endprodukt mit einem Gehalt von 83 % an Endstoff I in einer Ausbeute, bezogen auf Propylenoxid : 48 % der Theorie, bezogen auf Methylvinylether : 59 % der Theorie.

Der Endstoff I hat die folgende Zusammensetzung : 1,1,3,3-Tetramethoxypropan (8 %) vom Kp 30 °C/13 mbar ; 2-(2′,2′-Dimethoxyethyl)-4-methyl-(1,3)-dioxolan (64 %) vom Kp 37 °C/0,5 mbar ; Malondialdehyd-(bis-1,2-propylenacetal) (17 %) vom Kp 50 °C/0,4 mbar ; 1-Methoxypropoxy-1,3,3-trimethoxypropan (5 %) vom Kp 65 bis 70 °C/0,4 mbar ; 2-(2-Methoxy-2-methoxypropoxy-ethyl)-4-methyl-(1,3)-dioxolan (6 %) vom Kp 65 bis 70 °C/0,4 mbar.

## Beispiel 2

In einem Reaktor legt man 1 000 Teile Methylformiat und 2 Teile $BF_3 \cdot HCOOCH_3$ vor und erwärmt auf 30 °C. Dann wird innerhalb von 2 Stunden bei 30 bis 32 °C eine Mischung aus 110 Teilen 1,2-Propylenoxid und 50 Teilen Methylformiat zugegeben und das Reaktionsgemisch bis 70 °C Siedetemperatur eingeengt. Anschließend werden bei 20 bis 30 °C innerhalb von einer Stunde 58 Teile Methylvinylether eingeleitet. Nach Zugabe von 2 Teilen Tetramethylethylendiamin zur Neutralisation des Katalysators wird das Reaktionsgemisch wie in Beispiel 1 destilliert. Man erhält bei 25 bis 50 °C/25 mbar 46 Teile Vorlauf und bei 55 bis 85 °C/1 mbar 179 Teile roher Endstoff I (Reinheit 87,4 %) und einer Ausbeute, bezogen auf Propylenoxid : 46 % der Theorie, bezogen auf Methylvinylether : 87,4 % der Theorie.

Zusammensetzung des reinen Endstoffs :

| | |
|---|---|
| 4 % 1,1,3,3-Tetramethoxypropan Kp : | 30 °C/1,3 mbar |
| 76 % 2-(2',2'-Dimethoxyethyl)-4-methyl-1,3-dioxolan Kp : | 37 °C/0,5 mbar |
| 10 % Malondialdehyd(bis-1,2-propylen-acetal) Kp : | 50 °C/0,4 mbar |
| 5 % 1-Methoxypropoxy-1,3,3-Trimethoxypropan Kp : | 65 bis 70 °C/0,4 mbar |
| 5 % 2-(2'-methoxy-2'-methoxypropoxyethyl)-4-methyl-1,3-dioxolan | |

## Beispiel 3

Man verfährt wie in Beispiel 2, jedoch mit dem Unterschied, daß der Vorlauf aus diesem Versuch jetzt mit 78 Teilen Methylvinylether vermischt und diese Lösung in das Reaktionsgemisch gegeben wird. Nach Aufarbeitung erhält man 51 Teile Vorlauf und 232 Teile roher Endstoff I (Reinheit 88 %) in einer Ausbeute, bezogen auf Propylenoxid : 60 % der Theorie, bezogen auf Methylvinylether : 88 % der Theorie.

Die Zusammensetzung des Endstoffs entspricht der in Beispiel 2.

## Beispiel 4

In einen Reaktor werden kontinuierlich eingeleitet :

a) 1 110 Volumenteile pro Stunde Methylformiat,

b) 141 Teile Volumenteile pro Stunde einer 50-gewichtsprozentigen Lösung von Propylenoxid in Methylformiat und

c) 76 Volumenteile pro Stunde einer 0,8-gewichtsprozentigen Lösung von $BF_3$ in Methylformiat. Nach einer mittleren Verweilzeit von 30 Minuten bei 33 °C fließt das Reaktionsgemisch in eine Destillationsapparatur, in der bei 65 °C überschüssiges Methylformiat entfernt wird. Es werden dabei 1 110 Volumenteile pro Stunde Destillat erhalten, die in das erste Reaktionsgefäß zurückgeführt werden, und 217 Volumenteile pro Stunde Rückstand, der kontinuierlich in einen 2. Reaktor gepumpt wird. Gleichzeitig wird dieses Reaktionsgefäß mit 70 Volumenteilen pro Stunde einer 50-gewichtsprozentigen Lösung von Methylvinylether in Methylformiat beschickt. Das Reaktionsgemisch aus dem zweiten Reaktor fließt nach einer mittleren Verweilzeit von 30 Minuten bei 22 bis 26 °C in einen 3. Reaktor und wird dort während einer Reaktionsdauer von 5 Stunden gesammelt und mit insgesamt 5 Volumenteilen Tetramethylethylendiamin versetzt.

Die Destillation des Reaktionsaustrages entsprechend Beispiel 1 ergibt 630 Teile Methylformiat, 215 Teile Vorlauf und 346 Teile roher Endstoff I (Reinheit : 87 %) in einer Ausbeute, bezogen auf Propylenoxid : 33 %, bezogen auf Methylvinylether : 63 %.

Zusammensetzung des Endstoffs : wie in Beispiel 2.

## Beispiel 5

Man verfährt wie in Beispiel 4, jedoch mit dem folgenden Unterschied : die Destillation wird bei 73 °C durchgeführt, wobei 1 210 Volumenteile pro Stunde Destillat anfallen, die in das erste Reaktionsgefäß geleitet werden, und zum Lösen des Methylvinylethers wird jetzt der Vorlauf aus Beispiel 4 verwendet. Nach Aufarbeitung des Reaktionsaustrags erhält man 544 Teile Methylformiat, 248 Teile Vorlauf und 560 Teile roher Endstoff I (Reinheit : 85,2 %) in einer Ausbeute, bezogen auf Propylenoxid : 50 %, bezogen auf Methylvinylether : 70 %.

Zusammensetzung des Endstoffs: wie in Beispiel 1.

## Beispiel 6

Man verfährt wie in Beispiel 4, verwendet jedoch anstelle der Propylenoxid-Lösung 107 Volumenteile pro Stunde einer 50-gewichtsprozentigen Ethylenoxid-Lösung in Methylformiat. In diesem Beispiel werden nur 40 Volumenteile pro Stunde der Methylvinylether-Lösung eingesetzt. Nach analoger Aufarbeitung erhält man als Endstoff eine Mischung aus 1,1,3,3-Tetramethoxypropan, 2-(2',2'-Dimethoxyethyl)-(1,3)-dioxolan, Malondialdehyd-bis-(-ethylenacetal), 1-Methoxy-ethoxy-1,3,3-trimethoxypropan und 2-(2'-Methoxy-2'-methoxy-ethoxy-ethyl)-(1,3)-dioxolan in einer Zusammensetzung, die der in Beispiel 2 entspricht.

## Patentansprüche

1. Verfahren zur Herstellung von Gemischen der Verbindungen (Ia), (Ib) und (Ic)

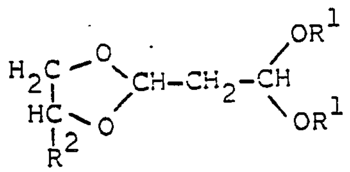

(Ia)

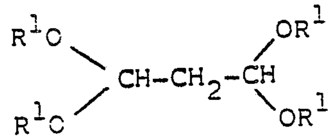

(Ib)

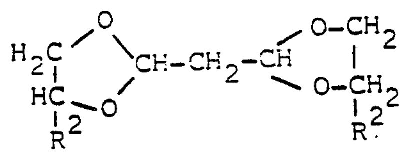

(Ic)

worin die einzelnen Reste $R^1$ gleich oder verschieden sein können und einen Alkylrest mit 1 bis 6 Kohlenstoffatomen bedeuten und $R^2$ Wasserstoff oder einen Alkylrest mit 1 bis 6 Kohlenstoffatomen darstellt, dadurch gekennzeichnet, daß man Alkylformiate der Formel (II)

$$HCOOR^1 \quad (II)$$

in Gegenwart katalytischer Mengen von Halogeniden des Bors und/oder Antimons (V) zunächst mit Oxiranen der Formel (III)

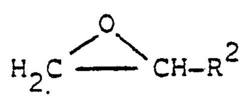

(III)

in der $R^2$ die vorgenannte Bedeutung hat, und dann in situ mit Alkylvinylethern der Formel (IV)

$$CH_2{=}CH{-}OR^1 \quad (IV)$$

in der $R^1$ die vorgenannte Bedeutung hat, umsetzt, wobei die Ausgangsstoffe (II) und/oder (III) in einem Verhältnis von 0,5 bis 3,0 mol pro Mol Ausgangsstoff (IV) verwendet werden.

2. Verfahren nach Anspruch 1, dadurch gekennzeichnet, daß man den Ausgangsstoff (III) in einem Verhältnis von 1 bis 2 mol pro Mol Ausgangsstoff (IV) verwendet.

## Claims

1. A process for the preparation of mixtures of compounds (Ia), (Ib) and (Ic)

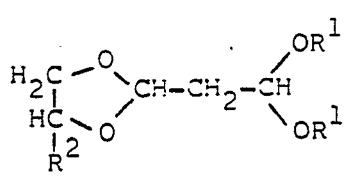

(Ia)

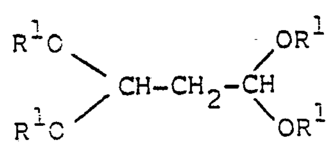

(Ib)

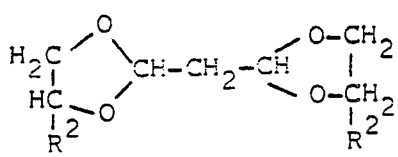

(Ic)

where the individual radicals $R^1$ may be identical or different and each is alkyl of 1 to 6 carbon atoms, and $R^2$ is hydrogen or alkyl of 1 to 6 carbon atoms, wherein an alkyl formate of the formula (II)

$$HCOOR^1 \qquad (II)$$

is first reacted with an oxirane of the formula (III)

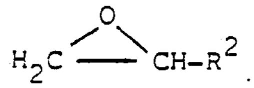

(III)

and then, in situ, with an alkyl vinyl ether of the formula (IV)

$$CH_2=CH—OR^1 \qquad (IV)$$

where $R^1$ has the above meaning, in the presence of a catalytic amount of a halide of boron and/or antimony (V), from 0.5 to 3.0 moles of starting materials (II) and/or (III) being used per mole of starting material (IV).

2. A process as claimed in claim 1, wherein from 1 to 2 moles of starting material (III) are used per mole of starting material (IV).

**Revendications**

1. Procédé de préparation de mélanges des composés (Ia), (Ib) et (Ic)

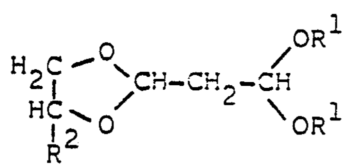

(Ia)

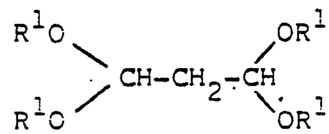

(Ib)

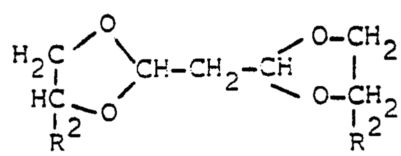

(Ic)

dans lesquelles les symboles $R^1$ individuels peuvent avoir des significations identiques ou différentes et représentent chacun un groupe alkyle possédant de 1 à 6 atomes de carbone et $R^2$ représente un atome d'hydrogène ou un groupe alkyle possédant de 1 à 6 atomes de carbone, caractérisé en ce que l'on fait d'abord réagir des formiates d'alkyle de la formule (II)

$$HCOOR^1 \qquad (II)$$

en présence de proportions catalytiques d'halogénures du bore et/ou de l'antimoine (V), sur des oxiranes de la formule (III)

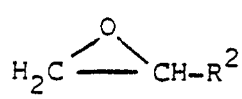

(III)

dans laquelle $R^2$ possède les significations qui lui ont été précédemment attribuées et ensuite, in situ, sur des éthers alkylvinyliques de la formule (IV)

$$CH_2=CH—OR^1 \qquad (IV)$$

dans laquelle $R^1$ possède les significations qui lui ont été précédemment attribuées, en utilisant les substances de départ (II) et/ou (III) en un rapport de 0,5 à 3,0 mole par mole de substance de départ (IV).

2. Procédé suivant les revendications 1, caractérisé en ce que l'on utilise la substance de départ (III). en un rapport de 1 à 2 moles par mole de la substance de départ (IV).